# EUROPEAN PATENT APPLICATION

(11) **EP 4 230 213 A1**
(43) Date of publication of application: **23.08.2023**
(21) Application number: 21880426.8
(22) Date of filing: 08.10.2021
(51) Int. Cl.: A61K 35/17, A61P 31/14, C12N 5/0783, A61K 38/21

(54) **ANTIVIRAL COMPOSITION COMPRISING NK CELLS AS ACTIVE INGREDIENT**

(30) Priority: 13.10.2020 KR 20200132050
(71) Applicant: Novocell Bio Co., Ltd., Incheon 22013 (KR)
(72) Inventor: MOON, Guy Young, Seongnam-si Gyeonggi-do 13458 (KR)
(74) Representative: Frenkel, Matthias Alexander
(86) International application number: PCT/KR2021/013915
(87) International publication number: WO 2022/080779

(57) **Abstract**

The present invention relates to an antiviral composition comprising Natural Killer (NK) cells as an active ingredient and, more particularly, to a composition for preventing or treating COVID-19 virus (SARS-CoV-2) infection comprising NK cells as an active ingredient and a method for culturing lymphocytes comprising anti-COVID-19 virus NK cells.

## Description

### Technical Field

The present invention relates to an antiviral composition comprising Natural Killer (NK) cells as an active ingredient and, more particularly, to a composition for preventing or treating COVID-19 virus (SARS-CoV-2) infection comprising NK cells as an active ingredient and a method for culturing lymphocytes comprising anti-COVID-19 virus NK cells.

### Background Art

In December 2019, pneumonia of unknown cause in the form of viral pneumonia began to occur in groups in Wuhan City, Hubei Province, China. Most of the patients had a history of visiting the Huanan Seafood Market, and chinese authorities officially announced on December 31, 2019 that pneumonia of unknown cause was prevalent in Wuhan. The Chinese Center for Disease Control and Prevention detected previously unknown new coronavirus from these pneumonia patients on January 7, 2020, and released the genetic information of the new coronavirus to the world on January 11, 2020. Such a virus has been named severe acute respiratory syndrome coronavirus 2 (SARS-CoV-2), and the World Health Organization has classified the disease caused by same as coronavirus disease 2019 (COVID-19), which refers to a disease caused by a coronavirus that occurred in 2019.

COVID-19 is transmitted when droplets (e.g., saliva droplets) from an infected person penetrate the respiratory tract or mucous membranes of one's eyes, nose or mouth. Once infected, after a culturing period of about 2 to 14 days (as estimated), fever (37.5 degrees), respiratory symptoms such as coughing and difficulty breathing, and pneumonia appear as the main symptoms, but there are also a few of cases of asymptomatic infection.

### Prior Art Citation

(Patent Document 0001) Korean Patent No. 2126783

### Summary of Invention

### Technical Problem

An object of the present invention is to provide a composition for preventing and treating COVID-19 virus (SARS-CoV-2) infection comprising a lymphocyte containing NK cells as an active ingredient, and a method for culturing lymphocytes comprising anti-COVID-19 virus NK cells, in order to solve the above-described problems of conventional technology in the art.

### Solution to Problem

The present invention provides an antivirus composition comprising NK cells as an active ingredient.

Specifically, the NK cells have the ability to kill coronavirus-infected cells, and, more specifically, the coronavirus is the COVID-19 virus (SARS-CoV-2).

Natural killer cells (NK cells) are important cells for innate immunity and are known to cause apoptosis by recognizing abnormal cells.

Immune cells comprising NK cells are cultured by the culturing method of the present invention and refer to cells comprising NK cells, NKT cells and T cells, for example, lymphocytes, and cells wherein the ratio of NK cells and NKT cells is 50% of the total ratio, for example, lymphocytes.

NKT cells or NK-like T cells are very similar cells to NK cells and, unlike NK cells that mature in a liver or bone marrow, mature in thymus and have a T cell receptor as a type of T cell. Both NK cells and NKT cells have NK cell receptors.

Specifically, the lymphocytes are characterized in that they have the ability to kill cells infected with COVID-19 virus (SARS-CoV-2).

The NK cells are activated NK cells, in which one or more of the activating receptors are overexpressed. The activating receptor may be any one of NKG2D, 2B4(CD244), DNAM-1(CD226), CD2, CD16, NKp30, NKp44, NKp46 and NKp80, but the present invention is not limited thereto.

After the lymphocytes are isolated from peripheral blood, they may be obtained by being cultured for 7 to 14 days, preferably for 7 to 9 days, and more preferably for 8 to 9 days. The present inventors verified the effect of lymphocytes cultured for 8 and 14 days on inhibiting the proliferation of COVID-19 virus (SARS-CoV-2), and it was confirmed that the lymphocytes cultured for 8 days showed a better effect on inhibiting the proliferation of the COVID-19 virus than the lymphocytes cultured for 14 days.

The lymphocytes may be proliferated and obtained by a method for proliferating NK cells comprising the step of (a) separating lymphocytes from peripheral blood and suspending them in a culture medium; (b) adding at least two kinds selected from the group consisting of IL-2, IL-15, IL-18, anti-CD56 antibody, anti-CD16 antibody, anti-NKp44 antibody, anti-NKp46 antibody and plasma to the suspension in step (a) and culturing them.

In addition, the method may further comprise the step of (c) after adding a new medium to the suspension in step (b) and suspending it, adding at least two kinds selected from the group consisting of IL-2, IL-15, IL-18, anti-CD56 antibody, anti-CD16 antibody, anti-NKp44 antibody, anti-NKp46 antibody and plasma to the suspension and culturing them.

In addition, the method may further comprise the step of (d) after adding a new medium to the suspension in step (c) and suspending it, adding at least two kinds selected from the group consisting of IL-2, IL-15, IL-18, anti-CD56 antibody, anti-CD16 antibody, anti-NKp44 antibody, anti-NKp46 antibody and plasma to the suspension and culturing them.

In addition, the method may further comprise the step of (e) adding only a new medium to the suspension in step (d) and culturing it.

Preferably, in steps (b) to (d), at least two kinds selected from the group consisting of IL-2, IL-15, IL-18, anti-CD56 antibody, anti-CD16 antibody, anti-NKp44 antibody, anti-NKp46 antibody and plasma may comprise at least any one of anti-NKp44 antibody and anti-NKp46 antibody. More preferably, all of IL-2, IL-15, IL-18, anti-CD56 antibody, anti-CD16 antibody, anti-NKp44 antibody, anti-NKp46 antibody and plasma may be added and cultured in steps (b) to (d).

In steps (b) to (d), 100 to 500 ng/mL of IL-2, 50 to 200 ng/mL of IL-15, 50 to 200 ng/mL of IL-18, 0.5 to 5 *µ*g/mL of anti-CD56 antibody, 0.5 to 5 *µ*g/mL of anti-CD16 antibody, 0.5 to 5 *µ*g/mL of anti-NKp44 antibody, and 0.5 to 5 *µ*g/mL of anti-NKp46 may be added, and 2 to 5 wt% of plasma relative to the total weight of the cell suspension may be added. Preferably, 200 ng/mL of IL-2, 80 ng/mL of IL-15, 80 ng/mL of IL-18, 3 *µ*g/mL of anti-CD56 antibody, 3 *µ*g/mL of anti-CD16 antibody, 3 *µ*g/mL of anti-NKp44 antibody, 3 *µ*g/mL of anti-NKp46 and 2.5 wt% of plasma may be added.

Preferably, in step (b), at least two kinds selected from the group consisting of IL-2, IL-15, IL-18, anti-CD56 antibody, anti-CD16 antibody, anti-NKp44 antibody, anti-NKp46 antibody and plasma may be added on the day of the start of culture.

Preferably, in step (c), at least two kinds selected from the group consisting of IL-2, IL-15, IL-18, anti-CD56 antibody, anti-CD16 antibody, anti-NKp44 antibody, anti-NKp46 antibody and plasma may be added on Day 2 of culture.

Preferably, in step (d), at least two kinds selected from the group consisting of IL-2, IL-15, IL-18, anti-CD56 antibody, anti-CD16 antibody, anti-NKp44 antibody, anti-NKp46 antibody and plasma may be added on Day 5 of culture, and in the step (e), a new medium may be added on Day 7.

Preferably, step (e) may further comprise adding a new medium and further culturing same for 1 to 2 days to obtain cultured lymphocytes. Therefore, the composition of the present invention comprises lymphocytes cultured for 8 to 9 days, more preferably for 8 days, as an active ingredient.

Referring to the experimental examples, the lymphocytes, specifically lymphocytes cultured for 8 to 9 days, may comprise 60 to 80% of NK cells, and 4 to 5% of NK-like T cells, and 10 to 11% of T cells. It was confirmed that the lymphocytes on Day 8 had a lower ratio of NK cells and significantly increased ratios of NK-like T cells and T cells, as compared to lymphocytes on Day 14 of culture.

In addition, the lymphocytes may have an IFN-γ secretion of 120 to 170 ng/ml, and it was confirmed that the IFN-γ secretion of the lymphocytes on Day 8 of culture similarly increased by at least 5 times as compared to that of the lymphocytes on the Day 14 of culture.

In another embodiment of the present invention, a method for producing lymphocytes containing activated NK cells for the prevention and treatment of COVID-19 virus (SARS-CoV-2) infection, that is, a method for culturing lymphocytes containing anti-COVID-19 virus (SARS-CoV-2) NK cells is provided.

The lymphocytes may be proliferated and obtained by a method for proliferating NK cells comprising the step of (a) separating lymphocytes from peripheral blood and suspending them in a culture medium; and (b) adding at least two kinds selected from the group consisting of IL-2, IL-15, IL-18, anti-CD56 antibody, anti-CD16 antibody, anti-NKp44 antibody, anti-NKp46 antibody and plasma to the suspension in step (a) and culturing them.

In addition, the NK cell proliferation method may further comprise the step of (c) after adding a new medium to the suspension in step (b) and suspending it, adding at least two kinds selected from the group consisting of IL-2, IL-15, IL-18, anti-CD56 antibody, anti-CD16 antibody, anti-NKp44 antibody, anti-NKp46 antibody and plasma to the suspension and culturing them.

In addition, the NK cell proliferation method may further comprise the step of (d) after adding a new medium to the suspension in step (c) and suspending it, adding at least two kinds selected from the group consisting of IL-2, IL-15, IL-18, anti-CD56 antibody, anti-CD16 antibody, anti-NKp44 antibody, anti-NKp46 antibody and plasma to the suspension and culturing them.

In addition, the NK cell proliferation method may further comprise the step of (e) adding only a new medium to the suspension in step (d) and culturing it.

Preferably, in steps (b) to (d), at least two kinds selected from the group consisting of IL-2, IL-15, IL-18, anti-CD56 antibody, anti-CD16 antibody, anti-NKp44 antibody, anti-NKp46 antibody and plasma may comprise at least any one of anti-NKp44 antibody and anti-NKp46 antibody.

Preferably, in step (b), at least two kinds selected from the group consisting of IL-2, IL-15, IL-18, anti-CD56 antibody, anti-CD16 antibody, anti-NKp44 antibody, anti-NKp46 antibody and plasma may be added on the day of the start of culture.

Preferably, in step (c), at least two kinds selected from the group consisting of IL-2, IL-15, IL-18, anti-CD56 antibody, anti-CD16 antibody, anti-NKp44 antibody, anti-NKp46 antibody and plasma may be added on Day 2 of culture.

Preferably, in step (d), at least two kinds selected from the group consisting of IL-2, IL-15, IL-18, anti-CD56 antibody, anti-CD16 antibody, anti-NKp44 antibody, anti-NKp46 antibody and plasma may be added on Day 5 of culture, and, in step (e), a new medium may be added on Day 7.

Preferably, step (e) may further comprise adding a new medium and further culturing same for 1 to 2 days to obtain cultured lymphocytes.

### Advantageous Effects of Invention

The lymphocytes cultured by the method for culturing lymphocytes comprising anti-COVID-19 virus NK cells selectively kill host cells infected with COVID-19 virus to produce the effect of inhibiting the proliferation of COVID-19 virus and, thus, can be usefully applied to a pharmaceutical composition for preventing or treating COVID-19 virus infection.

### Brief Description of Drawings

FIG. 1 is a graph showing the immune cell phenotypes of cultured cells on day 8 and day 14 of culture.
FIG. 2 shows the results of performing RT-PCR after co-cultivating the NK cells of the present invention with SARS-CoV-2-infected VeroE6 cells for 24 hours.

### Mode for Invention

Hereinafter, the present invention will be described in more detail through an embodiment. However, this embodiment is used only to help the understanding of the present invention, and the scope of the present invention is not limited to such an embodiment in any sense.

### Example: A method for producing immune cells comprising activated NK cells

### 1. Step 1

The inventors of the present invention first isolated lymphocytes from human peripheral blood to prepare immune cells containing activated NK cells. Specifically, 30 ml to 60 ml of human peripheral blood was collected by using a heparinized 10 ml vacuum collection tube (BD Vacutainer TM). Then, 15 ml of Ficoll-Paque Plus (endotoxin tested, density 1.077 g/ml, GE Healthcare, USA) solution was placed in a 50 ml lymphocyte separation tube (Leuco sep, Greiner Bio-One, Swiss) and centrifuged at 2,000 rpm such that the solution settled down the glass membrane in the tube. The collected blood was transferred to the separation tube and centrifuged at 2,500 rpm to separate the red blood cells and granulocyte layer to the lower layer, and the mononuclear cell layer (lymphocyte layer), platelets and plasma to the upper layer to separate the blood components.

The upper layer of plasma separated by centrifugation was inactivated in a 56°C water bath for 30 minutes. The lymphocyte layer was collected by using a sterilized pipette, collected in a 15 ml tube, and centrifuged to remove the supernatant. The lymphocytes from which the supernatant was removed were suspended in 10 ml of a buffer solution (PBS) and washed. The number of cells in a part of the suspension was counted by using a hemocytometer, and the suspension was centrifuged again to collect only lymphocytes. The number of cells of the harvested lymphocytes was determined to be 20 x 106 in total.

### 2. Step 2

After suspending the lymphocytes isolated in Step 1 in the culture medium (KBM502), 200 ng/mL of IL-2, 80 ng/mL of IL-15, 80 ng/mL of IL-18, 3 *µ*g/mL of anti-CD56 antibody, 3 *µ*g/mL of anti-CD16 antibody, 3 *µ*g/mL of anti-NKp44 antibody, 3 *µ*g/mL of anti-NKp46 antibody, and 2.5 wt% of plasma were added to the cell suspension, and immune cells were cultured in 25T flask and at 37°C for 1 to 2 days in a 5% CO₂ incubator. The antibodies and plasma in this step were added to the suspension on the day of the start of culture.

### 3. Step 3

After adding a new medium to the suspension in Step 2 where immune cells were being cultured and suspending it, 200 ng/mL of IL-2, 80 ng/mL of IL-15, 80 ng/mL of IL-18, 3 *µ*g/mL of anti-CD56 antibody, 3 *µ*g/mL of anti-CD16 antibody, 3 *µ*g/mL of anti-NKp44 antibody, 3 *µ*g/mL of anti-NKp46 antibody, and 2.5 wt% of plasma were added to the cell suspension, and cultured in a 75T flask and at 37°C for 2 to 3 days in a 5% CO₂ incubator. The antibodies and plasma in this step were added on Day 2 of culture.

### 4. Step 4

After adding a new medium to the suspension in Step 3 where immune cells were being cultured and suspending it, 200 ng/mL of IL-2, 80 ng/mL of IL-15, 80 ng/mL of IL-18, 3 *µ*g/mL of anti-CD56 antibody, 3 *µ*g/mL of anti-CD16 antibody, 3 *µ*g/mL of anti-NKp44 antibody, 3 *µ*g/mL of anti-NKp46 antibody, and 2.5 wt% of plasma were added to the cell suspension, and cultured in a 75T flask and at 37°C for 2 to 3 days in a 5% CO₂ incubator. The antibodies and plasma in this step were added on Day 5 of culture.

### 5. Step 5

### (1) Culturing method A

After adding 40 to 70 ml of a new medium to the suspension in Step 4 where immune cells were being cultured and suspending it, the immune cells were cultured in a 75T flask and at 37°C for 1 to 2 days in a 5% CO₂ incubator.

### (2) Culturing method B

IL-2 and plasma were added to the cell suspension in Step 4 where immune cells were being cultured, and the suspension was transferred to a 1,000ml CO₂ permeable bag, and the immune cells were cultured at 37°C for 7 to 10 days in a 5% CO₂ incubator. IL-2 and plasma in this step were added on Day 7 of culture.

### 6. Step 6

### (1) Culturing method A

In Step 5, the cell suspension in the 175T Flask was transferred to a centrifuge tube and centrifuged at 2,500 rpm to harvest the cells. The supernatant was discarded, and the pellet containing the cells was washed twice with 250 ml of sterile physiological saline and centrifuged to harvest the cells. The harvested cells were injected to a 100 ml pack of sterile saline solution for injection to complete the preparation and were used as cells on Day 8 of culture.

### (2) Culturing method B

In Step 5, the cell suspension in the CO₂ permeable bag was transferred to a centrifuge tube and centrifuged at 2,500 rpm to harvest the cells. The supernatant was discarded, and the pellet containing the cells was washed twice with 250 ml of sterile physiological saline and centrifuged to harvest the cells. The harvested cells were injected to a 100 ml pack of sterile saline solution for injection to complete the preparation and were used as cells on Day 14 of culture.

### Experimental Example 1. Analysis on the phenotype of immune cells containing prepared NK cells

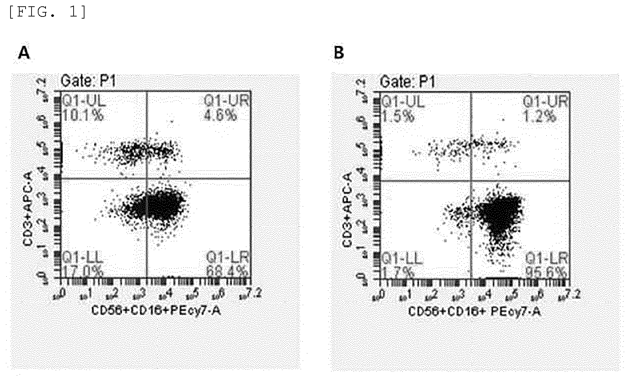

The inventors of the present invention cultured the immune cells comprising the NK cells cultured for 8 and 14 days, and confirmed the phenotypes of NK cells, NK-like T cells and T cells through flow cytometry *(see* FIG. 1).

**[Table 1]**

| | NK cells(%) | NK-like T cells(%) | T cells(%) |
|---|---|---|---|
| Day 7 | 68.4 | 4.6 | 10.1 |
| Day 14 | 95.6 | 1.2 | 1.5 |

Referring to [Table 1], as a result of analyzing the phenotypes by culturing lymphocytes on Day 8 of culture, the ratio of NK cells was 68.4%, the ratio of NK-like T cells was 4.6%, and the ratio of T cells was 10.1%. In addition, after culturing the isolated lymphocytes for 14 days, the ratio of NK cells was 95.6%, the ratio of NK-like T cells was 1.2%, and the ratio of T cells was 1.5%.

Thus, the inventors of the present invention confirmed that immune cells containing activated NK cells (in an amount of 50% or more) may be mass-produced by isolating lymphocytes from peripheral blood and culturing same. In addition, it was confirmed that, although the ratio of NK cells on Day 8 of culture was relatively lower than that on Day 14 of culture, the ratios of NK-like T cells and T cells on Day 8 of culture were higher than those on Day 14 of culture.

### Experimental Example 2. Confirmation of the amount of IFN-γ secretion, which is an indicator of the killing ability of immune cells containing NK cells prepared according to culturing stages

The inventors of the present invention divided immune cells containing NK cells cultured according to the culturing method into the cells on 8 days of culture and cells on 14 days of culture to verify the amount of IFN-γ secretion which shows NK cell activation.

After collecting all the cells on Day 8 and Day 14 of culture, centrifugation was performed at 1500 rpm for 20 minutes, and 1 ml of the supernatant was transferred to a new tube. The activities of the NK cells were verified by the amount of IFN-γ secretion by using human IFN-gamma ELISA kit (R&D systems).

**[Table 2]**

| Day of culture | IFN-γ (ng/ml) | | |
|---|---|---|---|
| | Case 1 | Case 2 | Case 3 |
| Day 7 | 128 | 168 | 120 |
| Day 14 | 19 | 24 | 21 |

Referring to [Table 2], it is confirmed that the NK cells on Day 8 of culture showed an increased amount of IFN-γ (cytokine) secretion, as compared to the immune cells containing NK cells on Day 14 of culture. As such, the NK cells on Day 8 of culture secreted the maximum amount of interferon-γ (IFN-γ) than the immune cells containing NK cells on Day 14 of culture and, thus, are regarded as having a very high activity of natural killer cells. Since the cells on Day 14 of culture were in the proliferation stage after the activation stage were finished, the cells have a relatively less ability to secrete IFN-γ, as compared to the activated cells on Day 8 of culture.

According to such results, the inventors of the present invention tested the effect of inhibiting the proliferation of COVID-19 virus by using NK cells cultured for 8 days.

### Experimental Example 3. Inhibitory effect of proliferation of COVID-19 virus through activated NK cells

### 1. RT-PCR experiment

Epithelial cells are infected with SARS-CoV-2. After a certain period of time, the virus is released out of the epithelial cells. The amount of this virus released to a cell culture medium can be quantitatively measured by real-time RT-PCR. The inventors of the present invention co-cultured VeroE6 cells uninfected with SARS-CoV-2 and VeroE6 cells infected with SARS-CoV-2, and NK cells for 24 hours, collected the culture medium from each group, and performed real-time-PCR.

VeroE6 cells infected with SARS-CoV-2 (target cell, T) were treated with immune cells (effector cells, E) on Day 8 of culture of Example at ratios of E:T=4:1 and 1:1. The genes of SARSCoV-2 (COVID-19) were identified as the following three genes in total: E, RdRp, and N genes.

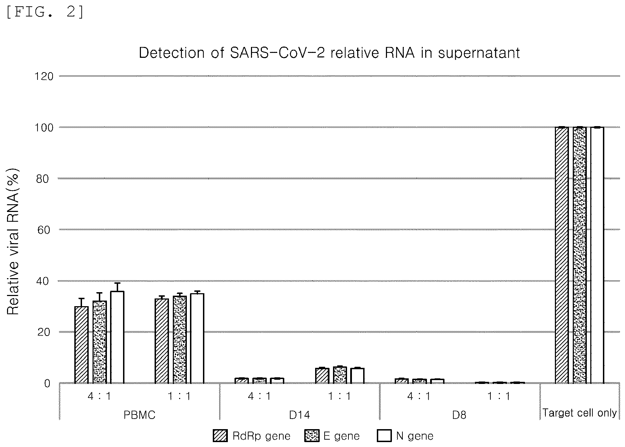

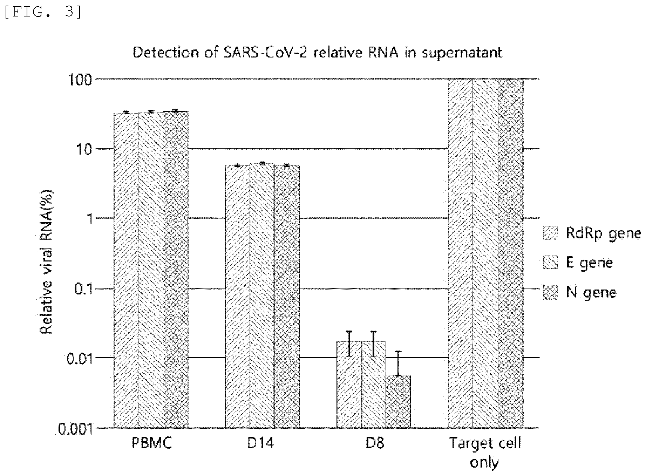

Engenomics' TOPreal^{™} One-step RT qPCR Kit (SYBR Green, low Rox) and 1 µL each of the primers shown in [Table 3] below were utilized in Real-time reverse transcription polymerase chain reaction (qRT-PCR). In terms of the conditions for reaction temperatures, reverse transcription was performed at 42 °C for 30 minutes, denaturation was performed at 95 °C for 10 minutes, and 35 cycles of the reaction at 95 °C for 30 seconds, 60 °C for 30 seconds, and 72 °C for 30 seconds were repeated, followed by additional stretching at 72 °C for 5 minutes. The results were observed and analyzed by using Biorad's CFX96 TouchTM Real-time PCR detection system and are shown in FIG 2.

**Table 3**

| Primer | Nucleotide sequence(5'-3') |
|---|---|
| E gene-F | ACAGGTACGTTAATAGTTAATAGCGT |
| E gene-R | FAM-ACACTAGCCATCCTTACTGCGCTTCG-BBQ |
| RdRp-F | ATATTGCAGCAGTACGCACACA |
| RdRp-R | GTGARATGGTCATGTGTGGCGG |
| N gene-F | FAM-CAGGTGGAACCTCATCAGGAGATGC-BBQ |
| N gene-R | FAM-CCAGGTGGWACRTCATCMGGTGATGC-BBQ |

Referring to FIG. 2, the virus was found in the infected VeroE6 group that was not co-cultured, and the amount of the viruses detected in the group cultured with immune cells (NOVONK) was relatively low in the experimental group wherein PBMC and NOVO-NK were co-cultured. In addition, the experimental group on Day 8 of culture showed a lower amount of the detected viruses than that in the experimental group on Day 14 of culture. Through this, it was confirmed that the cells infected with the viruses showed a decreased amount of the infected viruses as the activities of the NK cells increased.

Therefore, it was confirmed that, when VeroE6 cells infected with SARS-CoV-2 and NK cells are co-cultured, the virus-infected cells are killed by the NK cells and the RNA of the virus is eliminated, resulting in a decrease in the amount of the viruses detected in the co-cultured culture medium.

The cells infected with SARS-CoV-2 and SARS-CoV-2 RNA will be killed or eliminated by the NK cells, which suggests that the prognosis of patients infected with COVID-19 depends on cytotoxic activation of NK cells, and the NK cells can be used an autoimmune cell therapeutic agent in the future.

### Sub-conclusion

Conventional therapeutic agents with immune cells are used as a therapeutic agent by culturing the cells for 14 to 21 days, which is for mass proliferation. However, even if massively proliferated, the cultured cells are not regarded as exhibiting antiviral effects. The step of activating immune cells is important for achieving antiviral effects.

Although the NK cells on Day 14 of culture through activation and proliferation steps in sequence also showed good antiviral effects, the NK cells on Day 8 of culture which passed through activation and partial proliferation steps showed more excellent antiviral effects.

After the inventors of the present invention prepared cells through the activation and proliferation steps according to culturing stages and verify the antiviral effects, it was confirmed that the NK immune cells in the activation step of secreting excessive amounts of IFN-γ should be contained to exhibit a superior antiviral effect. The inventors of the present invention confirmed same through experiments and have completed the present invention.

## Claims

1. A composition for preventing or treating COVID-19 virus (SARS-CoV-2) infection comprising lymphocytes containing NK cells as an active ingredient.

2. The composition for preventing or treating COVID-19 virus infection of claim 1, wherein the lymphocytes have the ability to kill COVID-19 virus-infected cells.

3. The composition for preventing or treating COVID-19 virus infection of claim 1, wherein the lymphocytes are cultured by a culturing method comprising:
(a) separating the lymphocytes from peripheral blood and suspending them in a culture medium;
(b) adding at least two kinds selected from the group consisting of IL-2, IL-15, IL-18, anti-CD56 antibody, anti-CD16 antibody, anti-NKp44 antibody, anti-NKp46 antibody and plasma to the suspension in step (a) and culturing them;
(c) after adding a new medium to the suspension in step (b) and suspending it, adding at least two kinds selected from the group consisting of IL-2, IL-15, IL-18, anti-CD56 antibody, anti-CD16 antibody, anti-NKp44 antibody, anti-NKp46 antibody and plasma to the suspension and culturing them;
(d) after adding a new medium to the suspension in step (c) and suspending it, adding at least two kinds selected from the group consisting of IL-2, IL-15, IL-18, anti-CD56 antibody, anti-CD16 antibody, anti-NKp44 antibody, anti-NKp46 antibody and plasma to the suspension and culturing them; and
(e) adding only a new medium to the suspension in step (d) and culturing it.

4. The composition for preventing or treating COVID-19 virus infection of claim 3, wherein, in step (b), at least two kinds selected from the group consisting of IL-2, IL-15, IL-18, anti-CD56 antibody, anti-CD16 antibody, anti-NKp44 antibody, anti-NKp46 antibody and plasma are added on the day of the start of culture; in step (c), at least two kinds selected from the group consisting of IL-2, IL-15, IL-18, anti-CD56 antibody, anti-CD16 antibody, anti-NKp44 antibody, anti-NKp46 antibody and plasma are added on Day 2 of culture; in step (d), at least two kinds selected from the group consisting of IL-2, IL-15, IL-18, anti-CD56 antibody, anti-CD16 antibody, anti-NKp44 antibody, anti-NKp46 antibody and plasma are added on Day 5 of culture; and, in the step (e), a new medium is added on Day 7 and further cultured for 1 to 2 days to obtain lymphocyte cells.

5. The composition for preventing or treating COVID-19 virus infection of claim 1, wherein the lymphocytes are those cultured for 8 to 9 days.

6. The composition for preventing or treating COVID-19 virus infection of claim 1, wherein the lymphocytes comprise 60 to 80% of NK cells, 4 to 5% of NK-like T cells, and 10 to 11% of T cells.

7. The composition for preventing or treating COVID-19 virus infection of claim 1, wherein the lymphocytes have an amount of IFN-γ secretion of 120 to 170 ng/ml.

8. A method for culturing lymphocytes comprising anti-COVID-19 virus NK cells, comprising:
(a) separating the lymphocytes from peripheral blood and suspending them in a culture medium;
(b) adding at least two kinds selected from the group consisting of IL-2, IL-15, IL-18, anti-CD56 antibody, anti-CD16 antibody, anti-NKp44 antibody, anti-NKp46 antibody and plasma to the suspension in step (a) and culturing them;
(c) after adding a new medium to the suspension in step (b) and suspending it, adding at least two kinds selected from the group consisting of IL-2, IL-15, IL-18, anti-CD56 antibody, anti-CD16 antibody, anti-NKp44 antibody, anti-NKp46 antibody and plasma to the suspension and culturing them;
(d) after adding a new medium to the suspension in step (c) and suspending it, adding at least two kinds selected from the group consisting of IL-2, IL-15, IL-18, anti-CD56 antibody, anti-CD16 antibody, anti-NKp44 antibody, anti-NKp46 antibody and plasma to the suspension and culturing them; and
(e) adding only a new medium to the suspension in step (d) and culturing it.

9. The method for culturing lymphocytes comprising anti-COVID-19 virus NK cells of claim 8, wherein
in step (b), at least two kinds selected from the group consisting of IL-2, IL-15, IL-18, anti-CD56 antibody, anti-CD16 antibody, anti-NKp44 antibody, anti-NKp46 antibody and plasma are added on the day of the start of culture;
in step (c), at least two kinds selected from the group consisting of IL-2, IL-15, IL-18, anti-CD56 antibody, anti-CD16 antibody, anti-NKp44 antibody, anti-NKp46 antibody and plasma are added on Day 2 of culture;
in step (d), at least two kinds selected from the group consisting of IL-2, IL-15, IL-18, anti-CD56 antibody, anti-CD16 antibody, anti-NKp44 antibody, anti-NKp46 antibody and plasma are added on Day 5 of culture; and
in step (e), a new medium is added on Day 7 and further cultured for 1 to 2 days to obtain lymphocyte cells.
